Europäisches Patentamt

European Patent Office

Office européen des brevets

(11) Veröffentlichungsnummer: **0 221 401 B1**

(12) **EUROPÄISCHE PATENTSCHRIFT**

(45) Veröffentlichungstag der Patentschrift: **11.09.91**

(51) Int. Cl.5: **H01P 1/06, A61B 6/00**

(21) Anmeldenummer: **86114150.5**

(22) Anmeldetag: **13.10.86**

(54) **Rotierende Datenübertragungsvorrichtung.**

(30) Priorität: **25.10.85 DE 3538035**

(43) Veröffentlichungstag der Anmeldung:
**13.05.87 Patentblatt 87/20**

(45) Bekanntmachung des Hinweises auf die
Patenterteilung:
**11.09.91 Patentblatt 91/37**

(84) Benannte Vertragsstaaten:
**DE FR**

(56) Entgegenhaltungen:
**EP-A- 0 180 213**     **DE-A- 3 331 722**
**FR-A- 1 572 005**     **FR-A- 2 445 087**
**FR-A- 2 522 884**     **US-A- 4 427 983**

(73) Patentinhaber: **Siemens Aktiengesellschaft**
**Wittelsbacherplatz 2**
**W-8000 München 2(DE)**

(72) Erfinder: **Ermert, Helmut, Prof. Dr. Ing.**
**Eichenring 15**
**W-8551 Röttenbach(DE)**
Erfinder: **Ulherr, Thomas, Dipl.-Ing.**
**Königshammerstrasse 21**
**W-8500 Nürnberg 50(DE)**
Erfinder: **Bär, Albrecht, Dipl.-Ing.**
**Weiselstrasse 42**
**W-8520 Buckenhof(DE)**

**Beschreibung**

Die Erfindung betrifft eine Datenübertragungsvorrichtung zur Datenübertragung zwischen einem Rotor und einem Stator, bei der auf dem Rotor oder dem Stator eine Anzahl von Sendern angeordnet ist und auf dem Stator bzw. Rotor eine abweichende Anzahl von Empfängern angeordnet ist, wobei die jeweiligen Anzahlen so gewählt sind, daß eine kontinuierliche Datenübertragung zwischen Rotor und dem Stator erfolgt, und bei der zwischen den Sendern und den Empfängern eine ringförmige Datenübertragungsstrecke angeordnet ist.

In der DE-OS 28 46 526 ist eine Datenübertragungsvorrichtung dieser Art beschrieben, bei der der Rotor vom Drehkranz eines Computertomographen gebildet ist, auf dem ein Röntgenstrahler und ein Strahlendetektor angeordnet sind. Die Übertragung der vom Detektor erzeugten Daten erfolgt mit Hilfe eines um das Drehzentrum gekrümmten Ringes aus lichtleitendem Material, auf dessen Oberfläche eine Lichtquelle strahlt, die den zu übertragenden Signalen entsprechende Lichtsignale ausstrahlt. Der Ring leitet das eingestrahlte Licht über seinen gesamten Umfang weiter und weist eine Kopplungsstelle auf, an der ein Lichtempfänger am feststehenden Teil angeordnet ist.

Durch die DE-A-28 55 379 und die DE-A-33 31 722 sind weitere rotierende Datenübertragungsvorrichtungen für Computertomographen bekannt. Ferner ist es durch die US-A-4 427 983 bekannt, Daten von einem rotierenden ringförmigen Teil zu einem feststehenden ringförmigen Teil mit Hilfe von diskreten Anzahlen von Sendern und Empfängern zu übertragen. Zwischen dem rotierenden und dem feststehenden Teil breitet sich dabei ein gleichförmiges elektrisches Feld aus, über das die Sende- bzw. Empfangsenergie zu einer bzw. von einer Radarantenne übertragen wird. Eine Unterteilung der Datenübertragungsstecke in eine Anzahl von Einzelstrecken zur gleichzeitigen Übertragung mehrerer Informationen ist nicht vorgesehen und bei einer Radarantenne auch nicht erforderlich. Schließlich ist eine rotierende Datenübertragungsvorrichtung mit einem ringförmigen Hohlleiter und je einem Sender und einem Empfänger auch durch die DE-A-25 22 884 bekannt.

Der Erfindung liegt die Aufgabe zugrunde, eine rotierende Datenübertragungsvorrichtung der eingangs genannten Art so auszubilden, daß bei einfachem Aufbau eine störungsfreie Datenübertragung, insbesondere hoher Datenraten, gewährleistet ist, wobei mit einer einzigen Datenübertragungsstrecke mehrere Übertragungskanäle gebildet sind, in denen Informationen auch in beiden Richtungen übertragen werden können.

Diese Aufgabe ist erfindungsgemäß dadurch gelöst, daß die Datenübertragungsstecke symmetrisch in eine Anzahl von separaten Einzelstrecken unterteilt ist, und daß Sender und Empfänger so angeordnet sind, daß die gleichzeitige Übertragung mehrerer Information zwischen mehreren Sender-Empfänger-Paaren erfolgt.

Eine Ausbildung der Datenübertragungsvorrichtung, die sich insbesondere zur Anwendung in einem Computertomographen für die Übertragung hoher Datenraten eignet, wird dadurch erhalten, daß die Sender modulierte hochfrequente Wellen, z.B. elektromagnetischer Art, in den jeweils zugeordneten Wellenleiter einkoppeln, und daß die Empfänger zur Abnahme der in den Wellenleitern geführten Wellen ausgebildet sind. Mit Hilfe der sich im Wellenleiter ausbreitenden hochfrequenten Wellen wird dabei eine hohe Datenrate übertragen.

Die Erfindung ist nachfolgend anhand eines in der Zeichnung dargestellten Ausführungsbeispieles für in Hohlleitern geführte elektromagnetische Mikrowellen näher erläutert. Es zeigen:

Fig. 1     eine Prinzipdarstellung einer Datenübertragungsvorrichtung nach der Erfindung,

Fig. 2     eine detaillierte Darstellung der Datenübertragungsvorrichtung gemäß Figur 1, und

Fig. 3     das Schaltbild eines Computertomographen zur Erläuterung der Wirkungsweise der Datenübertragungsvorrichtung gemäß den Figuren 1 und 2.

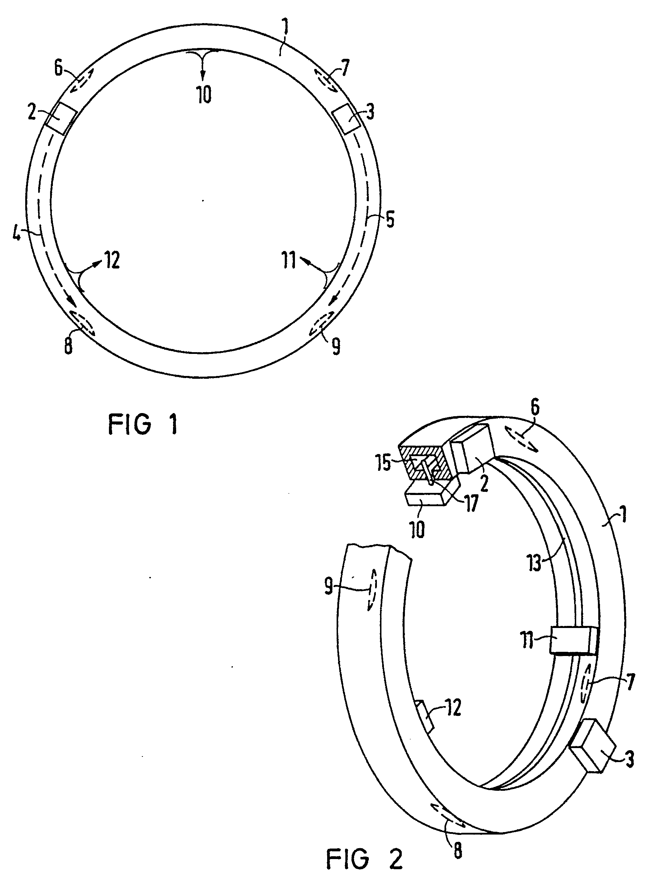

In der Figur 1 ist ein Hohlleiter 1 dargestellt, auf dem zwei Mikrowellensender 2, 3 befestigt sind. Die Mikrowellensender 2, 3 strahlen in der durch die Pfeile 4, 5 gekennzeichneten Richtung in den Hohlleiter 1. Vier Wellensümpfe 6 bis 9 teilen den Hohlleiter 1 in vier gleich lange Abschnitte. Bei dem Beispiel ist davon ausgegangen, daß der Hohlleiter 1 mit den Sendern 2, 3 rotiert und daß die Datenübertragung von den beiden Sendern 2, 3 zu drei um jeweils 120° gegeneinander versetzten, ortsfesten Empfängern 10, 11, 12 erfolgt. Dabei können die beiden anderen der vier durch die Wellensümpfe gebildeten Hohlleiterabschnitte für eine zweite Datenübertragungsstrecke genutzt werden.

Aufgrund der gewählten ungleichen Anzahl von zwei Sendern und drei Empfängern ist eine kontinuierliche Datenübertragung sichergestellt, da immer ein Sender und ein Empfänger miteinander kommunizieren. Insbesondere gilt hinsichtlich dieser Kommunikation unter der Voraussetzung, daß die gezeichnete Stellung des Hohlleiters 1 der Nullgrad-Stellung entspricht, für eine Drehung im Uhrzeigersinn folgende Zuordnung:

0° - 60° : 3 - 11

60° - 120° : 2 - 10

120° - 180° : 3 - 12

180° - 240° : 2 - 11
240° - 300° : 3 - 10
300° - 360° : 2 - 12

Die Verwendung eines einzigen Hohlleiters zur Datenübertragung von einem rotierenden zu einem feststehenden Teil ergibt einen sehr einfachen Aufbau, der sich zur Anwendung in einem Computertomographen eignet, wie er nachfolgend anhand der Figur 2 näher erläutert ist. Dabei erfolgt in der dargestellten Weise eine gerichtete Einkopplung mit Hilfe der beiden Sender 2, 3. Tote Winkel sind vermieden. Beim Umschalten zwischen den Sendern und Empfängern sind Phasensprünge vermieden, so daß sich die dargestellte Vorrichtung für die Übertragung hoher Datenraten eignet.

Die Figur 2 zeigt, daß der Hohlleiter 1 als Ring mit rechteckigem Querschnitt ausgebildet ist, welcher an seiner Innenseite einen Schlitz 13 aufweist. An einer Seitenfläche des Hohlleiters 1 sind die beiden Sender 2, 3 befestigt, die als gerichtete Koppler ausgeführt sind und durch die Seitenfläche des Hohlleiters 1 in den Innenraum 15 des Hohlleiters 1 strahlen.

Bei der Rotation des Hohlleiters 1 bewegt sich dieser relativ zu den drei feststehenden Empfängern 10, 11, 12, die mit Leitern, von denen der Leiter 17 des Empfängers 10 dargestellt ist, durch den Schlitz 13 in den Innenraum 15 des Hohlleiters 1 ragen und dort die jeweils sich ausbreitende Mikrowelle abtasten. Die Wellensümpfe 6, 7, 8, 9 können in bekannter Weise von angepaßten Absorberstrukturen im Innern des Hohlleiters 1 gebildet sein.

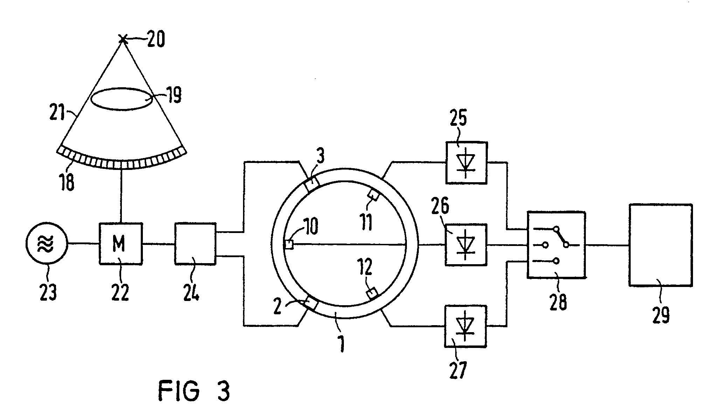

Die Figur 3 zeigt schematisch den Strahlendetektor 18 eines Computertomographen, der das einen im Querschnitt gezeigten Patienten 19 durchsetzende, vom Fokus 20 eines Röntgenstrahlers ausgehende Röntgenstrahlenbündel 21 erfaßt. Der Strahlendetektor 18 besteht aus einer Reihe von beispielsweise 512 Detektorelementen und die Daten dieser Detektorelemente werden einem Modulator 22 zugeführt, der die Wellen einer Mikrowellenquelle 23 den jeweils zu übertragenden Daten entsprechend moduliert. Über einen Verteiler 24 werden die modulierten Mikrowellen zu den beiden Sendern 2, 3 des Hohlleiters 1 geführt und werden von den Empfängern 10 bis 12 Demodulatoren 25, 26, 27 und von dort über einen Umschalter 28, z.B. einen Multiplexer, einem Rechner 29 zugeführt. Der Rechner 29 berechnet aus den übertragenen Daten, die bei der Rotation der Abtasteinheit 18, 20 um den Patienten 19 geliefert werden, ein Querschnittsbild des Patienten 19.

Bei dem Computertomographen gemäß Figur 3 umfaßt der rotierende Teil den Hohlleiter 1, mit dem der Röntgenstrahler sowie der Strahlendetektor 18 fest verbunden sind. Diese Teile sind in der Figur 3 lediglich der Übersichtlichkeit halber getrennt gezeichnet.

Bei dem in den Figuren 1 und 2 dargestellten Ausführungsbeispiel sind folgende Varianten möglich:

Anstelle von zwei Sendern und drei Empfängern können auch andere geeignete Anzahlen gewählt werden. Wesentlich ist jedoch, daß die jeweiligen Anzahlen der Sender und Empfänger sich um eins unterscheiden, so daß eine kontinuierliche Datenübertragung erfolgt. Mit einer Unterteilung des Hohlleiters durch vier Wellensümpfe in vier gleich lange Abschnitte lassen sich zwei Kanäle in einer Richtung oder je ein Kanal in je einer Richtung realisieren. Mit einer größeren Anzahl getrennter Hohlleiterabschnitte läßt sich bei entsprechend mehr Umschaltungen zwischen den Empfängern eine größere Anzahl von Datenstrecken unterbringen.

Es ist auch möglich, den Hohlleiter 1 mit den Sendern 2, 3 ortsfest anzuordnen und relativ zu ihm die Empfänger 10 bis 12 rotieren zu lassen.

**Patentansprüche**

1. Datenübertragungsvorrichtung zur Datenübertragung zwischen einem Rotor (1, 2, 3) und einem Stator, bei der auf dem Rotor (1, 2, 3) oder dem Stator eine Anzahl von Sendern (2, 3) angeordnet ist und auf dem Stator bzw. Rotor (1, 2, 3) eine abweichende Anzahl von Empfängern (10 bis 12) angeordnet ist, wobei die jeweiligen Anzahlen so gewählt sind, daß eine kontinuierliche Datenübertragung zwischen Rotor (1, 2, 3) und dem Stator erfolgt, und bei der zwischen den Sendern (2, 3) und den Empfängern (10 bis 12) eine ringförmige Datenübertragungsstrecke (1) angeordnet ist, **dadurch gekennzeichnet,** daß die Datenübertragungsstrecke (1) symmetrisch in eine Anzahl von separaten Einzelstrecken unterteilt ist, und daß Sender (2, 3) und Empfänger (10 bis 12) so angeordnet sind, daß die gleichzeitige Übertragung von Information zwischen mehreren Sender-Empfänger-Paaren erfolgt.

2. Datenübertragungsvorrichtung nach Anspruch 1, **dadurch gekennzeichnet,** daß die Sender (2, 3) in einen die ringförmige Datenübertragungsstrecke bildenden Hohlleiter (1) strahlen, daß sich die Strahlung im Hohlleiter (1) ausbreitet und daß die Empfänger (10 bis 12) zur Abnahme der Hohlleiterwellen ausgebildet sind.

3. Vorrichtung nach Anspruch 2, **dadurch gekennzeichnet,** daß ein einziger Hohlleiter (1) vorhanden ist, der zusammen mit den Sendern (2, 3) rotiert, wobei die Empfänger (10 bis 12) ortsfest angeordnet sind oder der zusammen mit den Sendern (2, 3) ortsfest ist, während die Empfänger (10 bis 12) rotieren.

4. Vorrichtung nach einem der Ansprüche 1 bis 3, **dadurch gekennzeichnet,** daß bei zwei Sendern (2, 3) drei Empfänger (10 bis 12) vorhanden sind.

5. Vorrichtung nach einem der vorhergehenden Ansprüche, **gekennzeichnet durch** die Anwendung bei einem Computertomographen (Fig. 3) zur Übertragung der Daten des rotierenden Strahlendetektors (18) zu einem feststehenden Teil (10 bis 12).

## Claims

1. A data transmission device for data transmission between a rotor (1,2,3) and a stator, in which a number of transmitters (2,3) are arranged on the rotor (1,2,3) or the stator and arranged on the stator or rotor (1,2,3) there is a variable number of receivers (10 to 12), wherein the respective numbers are selected such that a continuous data transmission between the rotor (1,2,3) and the stator occurs, and in which an annular data transmission link (1) is arranged between the transmitters (2,3) and the receivers (10 to 12), characterised in that the data transmission link (1) is divided symmetrically into a number of separate individual links and that the transmitters (2,3) and receivers (10 to 12) are arranged such that the simultaneous transmission of information occurs between several transmitter-receiver pairs.

2. A data transmission device according to claim 1, characterised in that the transmitters (2,3) radiate into a waveguide (1) forming the annular data transmission link, in that the radiation propagates in the waveguide (1), and in that the receivers (10 to 12) are constructed for accepting the waveguide waves.

3. A device according to claim 2, characterised in that an individual waveguide (1) is present, which rotates together with the transmitters (2,3), wherein the receivers (10 to 12) are arranged in a stationary manner, or which is stationary together with the transmitters (2,3), whilst the receivers (10 to 12) rotate.

4. A device according to one of claims 1 to 3, characterised in that with two transmitters (2,3) three receivers (10 to 12) are present.

5. A device according to one of the preceding claims, characterised by use in a computer tomograph (Figure 3) for transmitting the data of the rotating radiation detector (18) to a fixed part (10 to 12).

## Revendications

1. Dispositif de transmission de données pour réaliser la transmission de données entre un rotor (1,2,3) et un stator, et dans lequel un certain nombre d'émetteurs (2,3) sont disposés sur le rotor (1,2,3) ou le stator, et un nombre différent de récepteurs (10 à 12) sont disposés sur le stator ou le rotor (1,2,3), et dans lequel les nombres respectifs sont choisis de manière à obtenir une transmission continue de données entre le rotor (1,2,3) et le stator, et dans lequel une section annulaire de transmission de données (1) est disposée entre les émetteurs (2,3) et les récepteurs (10 à 12), caractérisé par le fait que la section de transmission de données (1) est subdivisée symétriquement entre un certain nombre de sections individuelles séparées et que les émetteurs (2,3) et les récepteurs (10 à 12) sont disposés de manière à obtenir la transmission simultanée d'informations entre plusieurs couples émetteur-récepteur.

2. Dispositif de transmission de données suivant la revendication 1, caractérisé par le fait que les émetteurs (2,3) rayonnent dans un guide d'ondes (1) constituant la section annulaire de transmission de données, que le rayonnement se propage dans le guide d'ondes (1) et que les récepteurs (10 à 12) sont agencés pour recevoir les ondes du guide d'ondes.

3. Dispositif suivant la revendication 2, caractérisé par le fait qu'il est prévu un seul guide d'ondes (1) qui tourne conjointement avec les émetteurs (2,3), les récepteurs (10 à 12) étant montés fixes, ou qui est monté fixe avec les émetteurs (2,3), tandis que les récepteurs (10 à 12) tournent.

4. Dispositif suivant l'une des revendications 1 à 3, caractérisé par le fait que dans le cas de deux émetteurs (2,3), il est prévu trois récepteurs (10 à 12).

5. Dispositif suivant l'une des revendications précédentes, caractérise par son utilisation, dans un tomodensitomètre assisté par ordinateur (figure 3), pour la transmission des données du détecteur rotatif de rayonnement (18) à une partie fixe (10 à 12).

FIG 1

FIG 2

FIG 3